# FASCICULE DE BREVET EUROPEEN

(11) **EP 3 134 101 B1**
(45) Date de publication et mention de la délivrance du brevet: **06.06.2018**
(21) Numéro de dépôt: 15715311.5
(22) Date de dépôt: 16.04.2015
(51) Int. Cl.: A61K 36/36, A61K 36/88, A61K 8/14, A61Q 19/00

(54) **UTILISATION DE CELLULES VEGETALES DE BOUGAINVILLIER POUR L'ENCAPSULATION D'INGREDIENTS ACTIFS**
VERWENDUNG VON BOUGAINVILLEA-PFLANZENZELLEN ZUM EINKAPSELN VON AKTIVEN INHALTSSTOFFEN
USE OF BOUGAINVILLEA PLANT CELLS FOR ENCAPSULATING ACTIVE INGREDIENTS

(30) Priorité: 23.04.2014 FR 1453647
(43) Date de publication de la demande: 01.03.2017
(73) Titulaire: Société De Recherche Cosmétique S.à.r.L., 2314 Luxembourg (LU)
(72) Inventeur: LECLERE, Jacques, F-45500 Saint-Gondon (FR); ENNAMANY, Rachid, F-33000 Bordeaux (FR)
(74) Mandataire: Peguet, Wilfried
(86) Numéro de dépôt international: PCT/EP2015/058241
(87) Numéro de publication internationale: WO 2015/162052

(56) Documents cités:
- EP-A1- 1 310 262
- EP-A2- 1 985 280
- WO-A2-2004/082643
- FR-A1- 2 949 975
- CHEN N ET AL: "Hyaluronic acid production promoter for use in topical preparation or cosmetics for moisturizing skin and preventing aging, contains plants e.g. Solena amplexicaulis, Laggera alata and/or Kalimeris indica, or its extract", WPI / THOMSON,, vol. 2007, no. 30, 5 avril 2007 (2007-04-05), XP002733987,
- CORNELIA SCHÜRCH ET AL: "Potential of plant cells in culture for cosmetic application", PHYTOCHEMISTRY REVIEWS, vol. 7, no. 3, 1 décembre 2007 (2007-12-01), pages 599-605, XP055043814, ISSN: 1568-7767, DOI: 10.1007/s11101-007-9082-0
- CHANCHAL DEEP ET AL: "Novel approaches in herbal cosmetics.", JOURNAL OF COSMETIC DERMATOLOGY JUN 2008, vol. 7, no. 2, juin 2008 (2008-06), pages 89-95, XP002734351, ISSN: 1473-2165

## Description

La présente invention se rapporte au domaine technique général des produits cosmétiques à usage topique, utilisables notamment sur la peau.

Plus précisément, la présente invention est relative à l'utilisation de cellules végétales dédifférenciées et élicitées de Bougainvillier pour l'encapsulation d'ingrédients actifs, et en particulier d'ingrédients actifs d'un point de vue cosmétique. Elle se rapporte également aux cellules végétales dédifférenciées et élicitées de Bougainvilliers encapsulant au moins un ingrédient actif, à leur procédé de préparation, aux compositions cosmétiques à application topique comprenant de telles cellules, à un procédé cosmétique non thérapeutique de traitement de la peau mettant en oeuvre une telle composition cosmétique, ainsi qu'à l'utilisation non thérapeutique d'une composition cosmétique à application topique comprenant de telles cellules pour le soin de la peau, et notamment pour améliorer la fermeté de la peau, notamment en favorisant la régénération du derme et de l'épiderme de la peau.

La peau est un véritable organe comprenant plusieurs couches intégrées, allant de la couche superficielle, l'épiderme, jusqu'aux couches plus profondes, le derme et l'hypoderme, et chacune de ces couches possède des propriétés spécifiques permettant à l'ensemble de réagir et de s'adapter aux conditions de son environnement.

L'épiderme, principalement composé de kératinocytes, de mélanocytes et des cellules de Langerhans, joue un rôle fondamental pour assurer la protection et le maintien d'une bonne trophicité. Les kératinocytes sont des cellules extrêmement dynamiques qui subissent une prolifération et une différenciation permanentes aux termes desquelles elles se transforment en cellules mortes (cornéocytes), s'éliminant régulièrement par desquamation.

Le derme se compose principalement de collagène, d'élastine et de protéoglycanes, molécules synthétisées par les fibroblastes dermiques. Les fibres de collagène assurent la résistance mécanique et la texture de la peau, l'élastine est responsable de l'élasticité, et les protéoglycanes, combinant protéine et glycosaminoglycane, jouent un rôle majeur de structure et d'hydratation de la peau. Les glycosaminoglycanes, tels que l'acide hyaluronique, sont des molécules très hygroscopiques qui sont capables de retenir des quantités importantes d'eau. D'autres cellules comme les macrophages et les leucocytes sont également présentes dans la couche du derme.

L'hypoderme, qui est la couche la plus profonde de la peau, contient les adipocytes qui produisent des lipides pour que le tissu sous-cutané fabrique une couche grasse protégeant les muscles, les os et les organes internes contre les chocs.

La peau est en renouvellement constant. La desquamation des cellules en surface est compensée par la naissance de nouvelles unités en profondeur. Lorsque les kératinocytes ont perdu leur noyau et leurs organites cellulaires, ils prennent le nom de cornéocytes. Avec les frottements, ils sont arrachés ou se décrochent sous l'action programmée d'enzymes spécifiques : c'est le phénomène de desquamation.

Les agressions de l'environnement, y compris les agressions climatiques telle que l'exposition au soleil, les variations de températures, la pollution, le stress, la fatigue, et le vieillissement en général ont tendance à altérer les capacités naturelles de régénération des kératinocytes composant l'épiderme qui devient alors moins dense, entrainant l'apparition de signes de vieillissement cutanés tels que rides et ridules et une fermeté diminuée.

Pour atténuer ou éliminer les signes du vieillissement de la peau, on a proposé divers traitements à base de compositions telles que crèmes et lotions contenant des alpha-hydroxyacides ou des rétinoïdes, appliquées régulièrement pour réduire progressivement le nombre de rides et ridules. On a aussi proposé des implants de collagène pour dissimuler les lignes d'expression autour des yeux ou de la bouche, la dermabrasion et les peelings chimiques pour éliminer la couche supérieure de la peau endommagée, la chirurgie esthétique, telle que la blépharoplastie (chirurgie des paupières) ou un lifting pour rajeunir une peau présentant un affaissement, ou encore une restructuration à l'aide d'un laser au dioxyde de carbone pour éliminer les ridules. Le brevet FR-A-2.783.169 décrit l'utilisation de pentapeptides du type Lys-Thr-Thr-Lys-Ser dans des compositions topiques pour favoriser la synthèse du collagène et des glycosaminoglycanes, et par conséquent la régénération cutanée.

Toutefois, si tous ces produits et méthodes connus peuvent avoir un effet favorable sur les signes du vieillissement cutané, par exemple en masquant ou en réduisant les rides, ils sont généralement sans incidence sur l'évolution cellulaire qui aboutit à ces signes du vieillissement.

Par ailleurs, les techniques d'encapsulation, apparues il y a plus de vingt ans, ne cessent de progresser pour optimiser la disponibilité et l'efficacité des produits actifs. L'encapsulation regroupe l'ensemble des technologies qui permettent la préparation de particules individualisées, constituées d'un matériau enrobant contenant un ingrédient actif. En cosmétique, l'encapsulation permet de protéger l'ingrédient actif des autres ingrédients de la formule, de le libérer lentement et de l'aider à pénétrer à travers les couches cutanées.

Les systèmes d'encapsulation présentent une grande variété de structures et peuvent donc être classés en deux grandes catégories que sont d'une part les systèmes matriciels également appelés microsphères et d'autre part les systèmes vésiculaires également appelés microcapsules.

Les microsphères sont des systèmes sphériques constitués d'une matrice (polymère, cire) dans laquelle est dispersé ou adsorbé un principe actif.

Les microcapsules sont des systèmes réservoirs composés d'une enveloppe piégeant un coeur liquide, solide ou gazeux. Par rapport aux microsphères, ces particules ont une grande capacité d'encapsulation et sont par contre plus fragiles et moins stables. En fonction des applications envisagées, l'enveloppe des microcapsules peut être choisie parmi les matériaux polymères (e.g. dérivés des acides lactiques et glycoliques (PLGA, PLA), éthyl-cellulose, poly-epsilon-caprolactone), les matériaux inorganiques (e.g. silice) ou bien encore les matériaux lipidiques dans le cas des liposomes formés d'une bicouche lipidique principalement constituée de phospholipides.

Les liposomes peuvent retenir plusieurs types de composés qu'ils soient hydrosolubles (encapsulés dans la phase aqueuse) ou liposolubles ou amphiphiles (empaquetés dans la bicouche lipidique). Les liposomes ont une structure très proche de celle des membranes cellulaires, ce qui leur permet de fusionner avec elles en libérant le ou les principes actifs qu'ils contiennent. Les liposomes sont utilisés dans le cas d'applications dermatologiques et cosmétiques, car ils permettent la délivrance cutanée voire transcutanée de molécules hydrophobes et/ou hydrophiles, sans limitation de leur masse moléculaire. Cependant, l'utilisation des liposomes ne donne pas toujours entièrement satisfaction en raison de la sensibilité à l'oxydation et l'instabilité chimique des phospholipides. Ils ne présentent par ailleurs en général pas d'activité intrinsèque puisqu'ils servent essentiellement à véhiculer un principe actif sans pour autant avoir eux-mêmes des propriétés pouvant être mises à profit pour le soin de la peau, en particulier pour améliorer la fermeté de la peau.

Les produits d'origine naturelle ou contenant des composés naturels sont par ailleurs de plus en plus appréciés par les consommateurs et consommatrices de produits cosmétiques. C'est pourquoi on trouve sur le marché des produits cosmétiques de plus en plus de produits contenant des substances naturelles ou d'origine naturelle.

La présente invention vise à fournir un nouvel ingrédient utilisable en cosmétique permettant de véhiculer et libérer de façon progressive au coeur de l'épiderme tout type d'ingrédient actif, qu'il soit hydrosoluble ou liposoluble, ne présentant pas les inconvénients des systèmes d'encapsulation connus de l'art antérieur et ayant en outre, en tant que tel, une activité intrinsèque pouvant avantageusement être mise à profit pour le soin de la peau, et en particulier pour favoriser la régénération du derme et de l'épiderme.

Ainsi, la présente invention a pour premier objet l'utilisation de cellules végétales dédifférenciées et élicitées de Bougainvillier pour l'encapsulation d'au moins un ingrédient actif, et en particulier d'au moins un ingrédient actif d'un point de vue cosmétique.

Elle a également pour objets :
- les cellules végétales dédifférenciées et élicitées de Bougainvilliers encapsulant au moins un ingrédient actif ;
- un procédé de préparation desdites cellules ;
- les compositions cosmétiques à application topique comprenant de telles cellules ;
- un procédé cosmétique non thérapeutique de traitement de la peau mettant en oeuvre une telle composition cosmétique, ainsi que
- l'utilisation non thérapeutique d'une composition cosmétique à application topique comprenant de telles cellules pour le soin de la peau, et notamment pour améliorer la fermeté de la peau, notamment en favorisant la régénération du derme et de l'épiderme de la peau.

En effet, les études effectuées par la société Demanderesse ont montré que des cellules dédifférenciées et élicitées de culture végétale de Bougainvillier, lorsqu'elles sont appliquées sur la peau, sont incorporées dans les différentes couches de l'épiderme et ont un effet positif sur la prolifération des cellules de l'épiderme, favorisant ainsi sa régénération et que ces mêmes cellules permettent en plus de véhiculer tout type d'ingrédients actifs et de les libérer de façon progressive au coeur des différentes couches du derme, que ces ingrédients actifs soient hydrosolubles ou liposoluble, ce qui en améliore l'efficacité. De plus, aux doses utilisées, les essais effectués ont montré que les compositions cosmétiques à base de cellules dédifférenciées de Bougainvillier utilisées dans la présente invention ne présentent aucune cytotoxicité.

Le Bougainvillier est un genre d'arbuste de la famille des *Nyctaginaceae.* Certaines espèces sont appelées bougainvillée (féminin) ou bougainvillier, (masculin) notamment *Bougainvillea glabra*, *Bougainvillea spectabilis* et *Bougainvillea buttiana.* Le premier spécimen de l'une de ces espèces a été découvert par le botaniste Philibert Commerson au Brésil, lors d'une expédition dirigée par l'explorateur français Louis Antoine de Bougainville dont il tire son nom. Les bougainvilliers sont des arbustes épineux grimpants aux vives couleurs qui contrairement aux apparences ne sont pas dues aux fleurs. Celles-ci sont petites et blanches, et ce sont les bractées de l'extrémité des rameaux qui entourent qui offrent des coloris variés rose, rouge, mauve, orange, jaune ou blanc. Ces arbustes sont largement utilisés comme plantes ornementales dans les régions tempérées chaudes, mais à ce jour, on ne lui connait pas d'application en cosmétique.

Toutes les espèces de Bougainvillier peuvent être utilisées selon l'invention, mais on peut plus particulièrement mentionner *Bougainvillea glabra*, *Bougainvillea alba*, *Bougainvillea spectabilis, Bougainvillea infesta,* et *Bougainvillea buttiana.*

Par cellules végétales dédifférenciées de Bougainvillier, on entend toute cellule végétale issue d'une culture *in vitro* de cellules de Bougainvillier, lesdites cellules ne présentant aucun des caractères d'une spécialisation particulière et étant capables de vivre par elles-mêmes et non en dépendance avec d'autres cellules.

Les cellules dédifférenciées et élicitées de Bougainvillier utilisables selon la présente invention peuvent être obtenues par des techniques connues de croissance ou multiplication cellulaire, en milieu liquide ou solide, de cellules de Bougainvillier cultivées *in vitro* dans un environnement contrôlé, dans des conditions aseptiques en l'absence de micro-organismes.

La technique de la culture végétale présente l'avantage de produire des cellules cultivées dans des conditions environnementales bien définies et reproductibles, en ce qui concerne notamment la température, la lumière et le milieu de culture, et de produire des cellules non différenciées se trouvant au même stade physiologique à un instant donné (dédifférenciées). Un autre avantage est la formation de métabolites secondaires en un temps plus court, de l'ordre de une à trois semaines, que chez la plante où ce délai est de plusieurs mois.

Par exemple, on peut préparer des cellules dédifférenciées et élicitées de Bougainvillier à partir d'explants de feuilles, de fleurs, de la tige ou de la racine.

Les cellules dédifférenciées et élicitées utilisables selon l'invention peuvent être obtenues par toute méthode connue de l'art antérieur telle que par exemple selon les méthodes décrites par E. F. George et P. D. Sherrington dans Plant Propagation by tissue culture, Handbook and Directory of Commercial Laboratories (Exegetics Ltd, 1984).

Les milieux de culture utilisables selon l'invention sont ceux généralement connus de l'homme du métier. On peut notamment citer à titre d'exemple les milieux de Gamborg, de Murashige Skoog, de Heller, de White, etc ... et dont la description complète peut être trouvée dans « Plant Culture Media : formulations and uses », E. F. George, DJM Puttock et H. J. George (Exegetics Ltd 1987, Tomes 1 & 2).

On entend par « élicitation », la mise en oeuvre d'un stress ou d'une agression (biologique, chimique ou physique) sur les cellules végétales dans leur milieu de culture afin de provoquer un ou plusieurs mécanismes de défense se concrétisant notamment, mais non uniquement, par la synthèse de métabolites secondaires d'intérêt tels que les phytoalexines.

Selon l'invention, l'élicitation des cellules dédifférenciées de Bougainvillier en milieu de culture peut être réalisée par application d'agents chimiques ou de stresses divers tels que pression, dépression, vide, variation de pression, présence d'un gaz, atmosphère variable, chaleur, froid, lumière, cycles de luminosité, radiations, toxines, agitation, contamination par des microorganismes (bactéries, levures, virus), ultrasons, rayonnement infra rouge ou ultraviolet, asphyxie, etc ...

Suivant la présente invention, on peut plus particulièrement utiliser des cellules dédifférenciées, élicitées et lyophilisées de Bougainvilliers. La lyophilisation des cellules permet d'améliorer leur conservation et, par suite, de faciliter leur incorporation dans les compositions cosmétiques.

Les éléments dosés dans les cellules lyophilisées sont les suivants (pour 100 g de cellules lyophilisées) :
**Protéines** (déterminée selon la méthode décrite dans l'Arrêté du 8 septembre 1977 relatif aux méthodes officielles d'analyse des produits diététiques et de régime) : 13,56 g
L'aminogramme de l'extrait protéique a mis en évidence les principaux acides aminés suivants. Dans ce qui suit, les teneurs en acides aminés sont indiquées en grammes pour 100 g de cellules lyophilisées.

| | |
|---|---|
| Acide aspartique | 10,61 |
| Acide glutamique | 10,15 |
| Proline | 6,75 |
| Lysine | 6,38 |
| Valine | 5,79 |
| Alanine | 5,29 |
| Sérine | 5,27 |
| Glycine | 4,82 |
| Arginine | 4,72 |
| Glutamine | 4,63 |
| Isoleucine | 4,61 |
| Leucine | 4,60 |
| Phénylalanine | 4,25 |
| Thréonine | 4,01 |
| Tyrosine | 3,70 |
| Asparagine | 3,29 |
| Tryptophane | 3,02 |
| Méthionine | 2,58 |
| Cystéine | 1,89 |
| Ornithine | 0,24 |

**Lipides** (déterminés selon la méthode décrite dans l'Arrêté du 8 septembre 1977 relatif aux méthodes officielles d'analyse des produits diététiques et de régime) : 1,08 g, se composant notamment de 73 % en masse environ d'acides gras saturés et insaturés en C₁₈ et de 21 % en masse environ d'acides gras saturés en C₁₆.

**Sucres** (déterminés par Chromatographie ionique - Détection ampérométrique pulsée (PAD)) :
- Glucose 11,23 g
- Fructose 13,10 g
- Lactose < 0,12 g
- Saccharose 10,78 g
- Maltose < 0,13 g

Les cellules dédifférenciées et élicitées de Bougainvillier utilisables selon l'invention se présentent sous la forme de vésicules comprenant une membrane végétale formée d'une bicouche lipidique délimitant un volume interne renfermant une phase encapsulée, généralement aqueuse. Elles sont caractérisées en ce qu'au moins un ingrédient actif est présent dans la phase encapsulée et/ou au sein de la bicouche lipidique. Les constituants de la membrane sont identiques aux lipides inter-cornéocytaires de la couche supérieure de l'épiderme et aux lipides intercellulaires (acides gras, céramides, cholestérol). Ainsi, après application des cellules dédifférenciées et élicitées conformes à l'invention sur la peau, le contenu cellulaire va pour diffuser par simple cisaillement de la peau ; le ou ingrédients actifs bénéficient d'une encapsulation naturelle et d'une libération par bio-mimétisme. Ainsi l'action des cellules s'effectue en 3 temps qui sont 1) la fragmentation des membranes lipidiques des cellules entières, 2) l'insertion dans les lipides inter-cornéocytaires et 3) la libération progressive du ou des ingrédients actifs par bio-mimétisme au sein même de la couche cornée.

Les cellules dédifférenciées et élicitées de Bougainvillier conformes à l'invention permettent ainsi d'encapsuler et de véhiculer vers la couche cornée de l'épiderme tout type d'ingrédient actif, qu'il soit liposoluble ou hydrosoluble.

En effet, et en fonction de leur affinité pour les milieux hydrophiles ou lipophiles, les ingrédients actifs auront soit tendance à s'incorporer dans les cellules végétales au niveau de la membrane lipidique s'ils sont liposolubles, ou bien dans la phase encapsulée, s'ils sont hydrosolubles.

Le ou les ingrédients actifs peuvent donc être choisis parmi tout type de composés habituellement utilisés dans les compositions cosmétiques. A titre d'exemple, on peut citer les extraits de plancton, les extraits d'origine végétale tels qu'un extrait de maca (qui a un effet sur la prévention de dérèglements liés à une déficience de la microcirculation cutanée et au stress oxydatif qui peut en résulter), un extrait de pétales de coquelicot agissant sur la nutrition cellulaire, un extrait de safran (qui a une action sur la régénération du derme et de l'épiderme), une combinaison d'extraits d'anchuse, de coquelicot et de passiflore qui, en association, ont un effet dans la prévention des signes du vieillissement cutané, un extrait de graines de mimosa, un extrait de pétales de souci, un extrait de barbitamao, un extrait de kigelia africana, un extrait d'avoine, etc ...

Selon une forme de réalisation particulièrement préférée de l'invention, l'ingrédient actif est choisi parmi les extraits végétaux parmi lesquels l'extrait de safran est tout particulièrement préféré. En effet, les essais réalisés par la Demanderesse ont montré que lorsque les cellules dédifférenciées et élicitées de Bougainvillier étaient utilisées pour encapsuler et véhiculer un extrait de safran, les effets de cet extrait étaient amplifiés, et ce dès 2 heures d'application.

Parmi les ingrédients actifs que l'on peut encapsuler dans les cellules de Bougainvillier conformément à l'invention, on peut également citer d'autres exemples tels que les vitamines anti-oxydantes telles que la vitamine E, par exemple l'acétate de tocophérol ou le tocotriénol, la vitamine C ; les polyphénols naturels ; l'hexylrésorcinol ; les agents anti-âge tels que par exemple un lipide comme le géranyl géranyl isopropanol (Juvinity®) qui réduit la formation des peroxydes intracellulaires et retarde ainsi le vieillissement cellulaire ou l'acétyl tetrapeptide-5 (CAS N° 820959-17-9).

La quantité en ingrédient actif encapsulé varie généralement de 0,01 à 5 % en masse par rapport à la masse totale des cellules dédifférenciées et élicitées de Bougainvillier, et encore plus préférentiellement de 0,1 à 3 % en masse.

Selon l'invention, la phase encapsulée est de préférence une phase aqueuse choisie parmi l'eau et les milieux de culture utilisés pour la culture *in vitro* desdites cellules.

Un autre objet de l'invention est un procédé de préparation de cellules végétales dédifférenciées et élicitées de Bougainvillier encapsulant au moins un ingrédient actif telles que décrites ci-dessus, ledit procédé comprenant les étapes suivantes :
1) la préparation de cellules dédifférenciées par culture *in vitro* en milieu de culture d'explants de Bougainvillier,
2) l'élicitation des cellules dédifférenciées obtenues à l'étape précédente dans le milieu de culture,
3) l'extraction des cellules dédifférenciées et élicitées obtenues ci-dessus à l'étape précédente,
ledit procédé étant caractérisé en ce qu'il comprend en outre une étape 4) d'encapsulation d'au moins un ingrédient actif dans des cellules dédifférenciées et élicitées, cette étape étant réalisée par incubation des cellules dédifférenciées et élicitées obtenues ci-dessus à l'étape 3) avec une solution ou une dispersion d'au moins un ingrédient actif dans un milieu liquide, ladite incubation.

L'étape 4) d'incubation est de préférence réalisée à une température inférieure ou égale à 30°C, et encore plus préférentiellement à une température variant de 20 à 30°C, pendant une durée de 12 à 48 heures environ.

Le milieu liquide utilisable pour effectuer l'incubation des cellules dédifférenciées et élicitées avec le ou les ingrédients actifs peut par exemple être choisi parmi les milieux utilisés à l'étape 1) du procédé pour la culture *in vitro* des cellules, c'est-à-dire pour la multiplication cellulaire des cellules de Bougainvillier.

A la fin de l'incubation, les cellules dédifférenciées et élicitées de Bougainvillier encapsulant au moins un ingrédient actif peuvent être récupérée dans le milieu liquide, par exemple par filtration.

Selon une forme de réalisation particulièrement avantageuse de l'invention, les cellules dédifférenciées et élicitées de Bougainvillier encapsulant au moins un ingrédient actif sont ensuite dispersées dans la glycérine pour obtenir une suspension de cellules, avant d'être incorporées dans une composition cosmétique, ceci afin d'améliorer la conservation des cellules au fil du temps.

Ainsi, selon une forme de réalisation préférée de l'invention, les cellules dédifférenciées et élicitées encapsulant au moins un ingrédient actif utilisable selon l'invention se présentent sous la forme d'une suspension desdites cellules dans la glycérine.

Ladite suspension de cellules dédifférenciées et élicitées de Bougainvillier dans la glycérine constitue ainsi un autre objet de l'invention. De préférence, la quantité de cellules dédifférenciées et élicitées de Bougainvillier au sein de ladite suspension varie de 15 à 30 % en masse environ, et encore plus préférentiellement est de l'ordre de 20 % en masse environ par rapport à la masse totale de ladite suspension.

L'invention a aussi pour objet une composition cosmétique à application topique caractérisée en ce qu'elle comprend, à titre d'ingrédient, des cellules végétales dédifférenciées et élicitées de Bougainvilliers encapsulant au moins un ingrédient actif, et au moins un support cosmétiquement acceptable.

De façon avantageuse, la composition cosmétique conforme à l'invention comprend de 0,001 à 10 % en masse d'une suspension de cellules végétales dédifférenciées et élicitées de Bougainvillier dans la glycérine et telle que décrite ci-dessus, par rapport à la masse totale de la composition cosmétique, et encore plus préférentiellement de 0,1 à 5 % en masse.

Au sens de la présente invention, on entend par « support cosmétiquement acceptable », les milieux comprenant de l'eau, un mélange d'eau et d'un ou plusieurs solvants organiques, ou un solvant ou un mélange de solvants organiques cosmétiquement acceptables.

Les compositions cosmétiques selon l'invention peuvent contenir, outre les cellules végétales dédifférenciées et élicitées de Bougainvillier encapsulant au moins un ingrédient actif, un ou plusieurs ingrédient actifs secondaires renforçant ou complétant avantageusement leur activité, et compatibles, c'est-à-dire non susceptibles de réagir les uns avec les autres ou de masquer ou limiter leurs effets. De tels ingrédients actifs peuvent par exemple être choisis parmi les ingrédients actifs pouvant être encapsulés dans les cellules végétales de Bougainvillier et tels que mentionnés précédemment.

Les compositions cosmétiques conformes à la présente invention peuvent se présenter sous les formes galéniques classiquement utilisées pour une application topique, c'est-à-dire sous forme de gel, d'émulsion (en particulier crème ou lait), d'émulsion biphasique huile-dans-eau ou eau-dans-huile, d'huile corporelle, de masque, de pommade, d'onguent, de lotion, de solution concentrée, lesdites formes galéniques contenant en outre des excipients et supports usuels et acceptables sur le plan cosmétologique. Elles sont utilisées de préférence sous forme de crèmes, de sérums, de lotion ou de gel.

Ces formes d'administration par voie topique sont préparées par les techniques usuelles, et par exemple, dans le cas d'une crème, par dispersion d'une phase grasse dans une phase aqueuse pour obtenir une émulsion huile-dans-eau, ou inversement pour préparer une émulsion eau-dans-huile. Dans le cas de crèmes, on peut utiliser des émulsions à structure lamellaire obtenues avec des lécithines hydrogénées ou des sucro-esters, contenant peu de produits éthoxylés ou n'en contenant pas du tout.

Les compositions topiques selon l'invention peuvent comprendre des excipients appropriés pour une application topique externe, en particulier des excipients acceptables sur le plan cosmétologique. Ces excipients appropriés pour la formulation sont bien connus de l'homme du métier et comprennent en particulier des agents de pénétration tels que le phytantriol, l'octyl dodécanol et l'escine ; les épaississants tels que les gommes naturelles et les polymères de synthèse ; les tensioactifs ; les émollients tels que l'octanoate de cétéaryle, le myristate d'isopropyle, l'isononanoate de cétéaryle, la diméthicone, la cyclométhicone, le 3-diisostéarate de polyglycéryle, le polyisobutène hydrogéné, l'alcool cétylique, le palmitate cétylique, le phosphate cétylique ; les émulsionnants tels que des dérivés de polyglycérol ; les conservateurs tels que le phénoxyéthanol et l'acide déhydroacétique ; les colorants ; les parfums ; etc...

Comme autres ingrédients utilisables dans les compositions de l'invention, on peut citer les agents hydratants tels que la glycérine, le butylène glycol, le sel de sodium de l'acide pyrrolidone carboxylique (sodium PCA), ainsi que des associations de dérivés glucosiques à effet hydratant et restructurant comme le produit Aquaxyl® (xylitylglucoside et anhydroxylytol et xylitol), et également les vitamines antioxydantes.

On peut aussi ajouter à la composition des glucides choisis principalement parmi le glucose, le glycogène et le tréhalose, ou encore un palmitoyl pentapeptide-3 tel que le Matrixyl® ou des dérivés tels que le palmitoyl GHK (possédant la chaîne Glycyl-Histidyl-Lysine) et le palmitoyl GQPR (Glycyl-Glutamyl-Prolyl-Arginine) ou le palmitoyl VGVAPG (Valyl-Glycyl-Valyl-Alanyl-Prolyl-Glycine) associé à un céramide-2, ainsi que d'une manière générale toute association avec un céramide.

On peut également ajouter à la composition des agents de protection contre les rayons ultraviolets, et par exemple des filtres solaires UV-A et UV-B hydrophiles ou lipophiles, ou des pigments formant écran anti-ultraviolet.

Les filtres solaires peuvent être choisis par exemple parmi la benzophénone ou un dérivé de benzophénone tel que la 2-hydroxy-4-méthoxy-benzophénone (Eusolex® 4360), ou un ester d'acide cinnamique et plus particulièrement le méthoxycinnamate d'octyle (Eusolex® 2292), le méthoxycinnamate d'éthyl-2-hexyle (Parsol MCX®), ou encore un cyano-β,β-diphénylacrylate tel que l'octocrylène (Eusolex® OCR), le 4-méthylbenzylidène camphre (Eusolex 6300®), et des dérivés du dibenzoylméthane tels que le 4-isopropyl dibenzoylméthane (Eusolex 8020), le t-butyl-méthoxy dibenzoylméthane (Parsol 1789®), et le 4-méthoxy-dibenzoylméthane.

Les pigments peuvent être choisis par exemple parmi le dioxyde de titane, l'oxyde de zinc et l'oxyde de zirconium.

Les compositions cosmétiques à application topique de l'invention permettent d'améliorer la pénétration des ingrédients actifs au sein de la couche cornée de l'épiderme et ainsi d'améliorer leur efficacité. Les cellules dédifférenciées et élicitées de Bougainvillier ont par ailleurs un effet positif intrinsèque sur la prolifération des cellules du derme et de l'épiderme qui peut avantageusement être combiné à l'effet du ou des ingrédients actifs qu'elles encapsulent.

Ainsi, l'invention a donc également pour autre objet, un procédé cosmétique non thérapeutique pour les soins de la peau, en particulier pour améliorer la fermeté de la peau en favorisant notamment la régénération du derme et de l'épiderme de la peau, caractérisé en ce qu'il consiste à appliquer sur les zones de la peau concernées au moins une composition cosmétique telle que définie ci-dessus, c'est-à-dire une composition cosmétique topique contenant une quantité efficace de cellules végétales dédifférenciées et élicitées de Bougainvillier encapsulant au moins un ingrédient actif.

Enfin, l'invention a pour objet l'utilisation non thérapeutique d'une composition cosmétique à application topique comprenant des cellules végétales dédifférenciées et élicitées de Bougainvillier encapsulant au moins un ingrédient actif pour les soins de la peau, en particulier pour améliorer la fermeté de la peau, notamment en favorisant la régénération du derme et de l'épiderme de la peau.

L'utilisation cosmétique de la composition cosmétique conforme à l'invention comprend tous les soins du corps et de la peau y compris les produits solaires, protecteurs et bronzants, les produits anti-âge, anti-séborrhéiques, toniques, les produits assurant l'amélioration de l'aspect de la peau y compris le traitement acnéique et les rougeurs cutanées.

Les exemples suivants illustrent l'invention plus en détail sans en limiter la portée. Dans tous les exemples de compositions qui suivent, les parties sont exprimées en masse, sauf indication contraire.

### EXEMPLES

### EXEMPLE 1 : Préparation d'une suspension de cellules dédifférenciées et élicitées de Bougainvillier

### 1. Prélèvement et culture des cellules dédifférenciées de Bougainvillier

Des morceaux de feuilles d'environ 5 à 10 mm ont été prélevés sur des feuilles fraîches de bougainvillier (*Bougainvillea glabra*).

Les morceaux de feuilles ont ensuite été stérilisés, puis mis en culture dans des boites stériles à température ambiante sur un milieu nutritif synthétique (milieu de Murashige et Skoog gélosé), jusqu'à formation, au niveau des explants, d'amas cellulaire dédifférenciées, appelés cals ou cellules mères.

Les cellules mères ont été prélevées au bout de 30 jours, puis repiquées sur un milieu nutritif neuf dans les mêmes conditions de culture que précédemment jusqu'à obtention de cals friables.

On a ainsi obtenu une collection de souches dédifférenciées stables présentant des caractéristiques de croissance et de production de métabolites.

### 2. Elicitation des cellules obtenues ci-dessus à l'étape précédente

L'élicitation des cellules a été réalisée par irradiation par la lumière UV à 180 nm pendant 2 heures, à l'aide d'une lampe Wilbert-Lourmat T-30C (600 µW/m²), positionnée à une distance de 1 m en éclairage direct au-dessus des cellules.

### 3. Préparation de la suspension cellulaire

Après l'élicitation, un morceau de cal a été prélevé puis mis en suspension dans un milieu nutritif liquide (milieu de Murashige et Skoog sans gélose).

A la fin de la culture (15 jours), les cellules ont été filtrées pour éliminer le milieu de culture puis rincées à l'eau froide (4°C). On a ainsi obtenu une biomasse fraîche d'environ 250 g de cellules par litre de culture.

Les cellules ont ensuite été mises en suspension dans de la glycérine végétale, à raison de 20 g de cellules pour 80 g de glycérine végétale, soit 20 % en masse. On a obtenu une suspension de couleur beige-marron.

### EXEMPLE 2 : Mise en évidence de l'absence de cytotoxicité de la suspension de cellules végétales de Bougainvillier préparée à l'exemple 1

Dans cet exemple on a testé la cytotoxicité éventuelle de la suspension de cellules dédifférenciées et élicitées de *Bougainvillea glabra* telle qu'obtenue ci-dessus par le procédé de l'exemple 1.

L'essai a été réalisé *in vitro* sur épidermes humains reconstruits (« *Reconstructed Human Epidermis* », RHE) modèle RHEps, de surface 0,5 cm², vendus par la société SkinEthic ®.

La suspension glycérinée de cellules obtenue ci-dessus à l'exemple 1 a été testée après dilution à 0,5 % dans avec du milieu de culture Murashige et Skoog non gélosé en application topique pendant 24 heures sur les épidermes, à raison de 2 µl par cm².

Les images histologiques (non représentées), obtenues après coloration à l'hématéine/éosine/safran (HES), des épidermes traités par la suspension étaient comparables à celles des épidermes témoins n'ayant reçu aucun traitement.

Ces résultats sont significatifs d'une absence de toxicité cutanée.

### EXEMPLE 3 : Préparation d'une suspension de cellules dédifférenciées et élicitées de Bougainvillier encapsulant un extrait de Safran

Dans cet exemple on a préparé une suspension de cellules dédifférenciées et élicitées de Bougainvillier encapsulant un extrait de fleurs Safran (Crocus sativus) à titre d'ingrédient actif.

L'extrait de safran a été obtenu à partir de fleurs de safran après prélèvement des stigmates. Les fleurs sans stigmates ont été séchées, réduites en poudres puis extraites à l'eau par macération.

Des cellules dédifférenciées et élicitées de Bougainvillier telles que préparées ci-dessus à l'exemple 1 ont été mises en suspension dans du milieu nutritif liquide (milieu de Murashige et Skoog sans gélose) contenant 1 % en masse d'extrait de Safran. L'incubation des cellules de Bougainvillier a été réalisée pendant 48 heures, à une température de 25°C afin d'encapsuler l'extrait de safran dans la phase aqueuse des cellules.

A la fin de la période d'incubation, les cellules de Bougainvilliers ont été récupérées par filtration puis mises en suspension dans la glycérine à raison de 20 % en masse pour 80 % en masse de glycérine.

On a ainsi obtenu une suspension comprenant 80 % en masse de glycérine, 20 % en masse de cellules dédifférenciées et élicitées de Bougainvillier encapsulant l'extrait de Safran.

### EXEMPLE 4 : Mise en évidence de l'incorporation des cellules dédifférenciées de Bougainvillier dans les différentes couches de l'épiderme

Dans cet exemple on a étudié l'aptitude des cellules dédifférenciées et élicitées de Bougainvillier à pénétrer dans les différentes couches de l'épiderme afin d'y délivrer l'ingrédient actif encapsulé.

### 4.1. Principe du test

Le principe du test est basé l'utilisation de la protéine verte fluorescente (connue également sous la dénomination anglophone « Green Fluorescent Protein » ou GFP), afin de visualiser le passage des cellules de Bougainvillier dans les différentes couches de l'épiderme.

### 4.2. Protocole

5 mM de GFP ont été ajoutés à la suspension de cellules végétales de *Bougainvillea glabra* préparée ci-dessus à l'exemple 1. Le mélange a été incubé pendant 4 heures à 37°C dans une atmosphère contenant 5 % de CO₂.

Une composition à 5 mM de GFP dans du milieu de culture Murashige et Skoog non gélosé a également été préparée.

L'étude a été réalisée sur des épidermes humains reconstruits *in vitro,* de modèle RHEps, fabriqués par la société SkinEthic. Ils ont été répartis en 3 lots (n=4, c'est-à-dire 4 épidermes par lot) :
**Lot 1** : 4 épidermes non traités ;
**Lot 2** : 4 épidermes traités par la GFP seule ;
**Lot 3** : 4 épidermes traités avec des cellules végétales dédifférenciées et élicitées de Bougainvillier incubées avec la GFP.

A réception, les épidermes ont été transférés dans des plaques de 12 puits contenant du milieu de culture pour épiderme vendu par la société SkinEthic (0,5 ml/puits) et placés à l'incubateur à 37°C, dans une atmosphère à 5% de CO₂ saturée d'humidité, pendant 24 heures avant de procéder au traitement des tissus.

2 µl/cm² de chaque échantillon à tester (Solution de GFP seule ou Suspension de cellules incubées avec la GFP) ont été appliqués en topique sur les épidermes (surface des épidermes : 0,5 cm²). Après traitement, les épidermes ont été replacés à l'étuve à 37°C et incubés pendant 2 heures (2 épidermes par lot) ou pendant 12 heures (2 épidermes par lot) en atmosphère air/CO₂ (95%/5% : v/v).

Après incubation, les épidermes ont été fixés dans une solution de formaldéhyde à 10 % puis inclus dans des blocs de paraffine. Des coupes verticales de 4 µm ont été réalisées au microtome, puis observées et photographiées sous un microscope optique de fluorescence (Zeiss).

### 4.3. Résultats

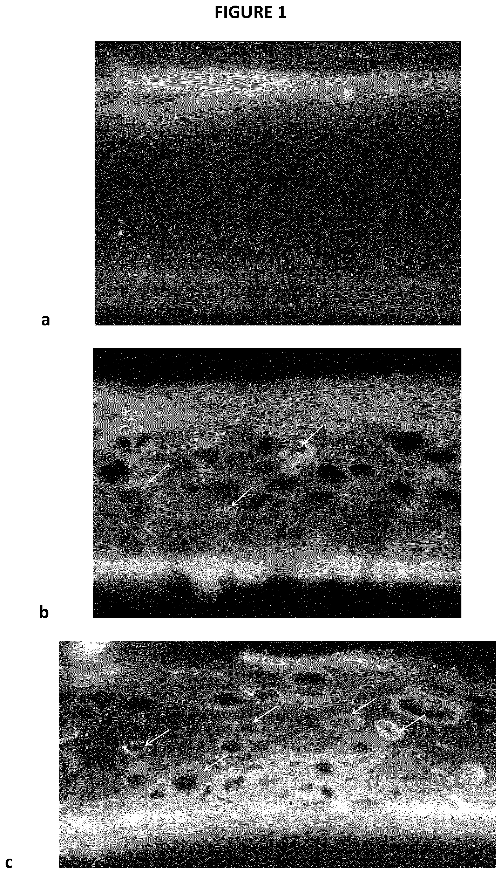

Les résultats obtenus sont reportés sur la figure 1 annexée qui donne une photographie d'un épiderme traité par la solution de GFP seule après 12 heures d'incubation (figure la), la photographie d'un épiderme traité pendant 2 heures avec la suspension de cellules dédifférenciées de Bougainvillier préalablement incubées avec la GFP (figure 1b), ainsi que la photographie d'un épiderme traité pendant 12 heures avec la suspension de cellules dédifférenciées de Bougainvillier préalablement incubées avec la GFP (figure 1c). Sur ces figures, les flèches blanches indiquent les cellules de bougainvilliers rendues fluorescence par la GFP et leur incorporation dans la couche de l'épiderme.

Ces résultats montrent que le traitement des épidermes par la solution de GFP seule n'induit aucun passage de cette dernière, aucun marquage fluorescent n'étant observé au sein des différentes couches de l'épiderme, la majorité de la fluorescence étant concentrée dans la couche cornée (figure la).

Par contre, concernant les épidermes traités avec la suspension de cellules dédifférenciées et élicitées de Bougainvillier préalablement incubées avec la GFP pendant 2 heures (figure 1b), on observe un passage de la protéine. En effet, un marquage fluorescent est observé au sein de l'épiderme, ce qui témoigne de la présence de la GFP et par conséquent de l'incorporation des cellules végétales dans les différentes couches de l'épiderme (couche granuleuse et couche spineuse).

Le traitement des épidermes pendant 12 heures avec la suspension de cellules dédifférenciées et élicitées de Bougainvillier préalablement incubées avec la GFP (figure 1c) conduit également à un marquage fluorescent, celui-ci étant observé au sein de toutes les couches, ce qui est significatif de l'incorporation des cellules dédifférenciées et élicitées de Bougainvillier aussi bien au niveau des couches granuleuse et spineuse que de la couche basale.

Ainsi, cet exemple démontre que les cellules dédifférenciées préparées selon l'invention peuvent pénétrer au travers des différentes couches de l'épiderme pour atteindre la couche basale et y véhiculer un ingrédient actif encapsulé par analogie à l'encapsulation à la GFP.

### EXEMPLE 5 : Evaluation de l'effet des cellules dédifférenciées et élicitées de Bougainvillier encapsulant un extrait de safran sur la régénération de l'épiderme

### 5.1 Principe du test

Le test est basé sur l'évaluation immunohistologique de l'index de prolifération Ki-67 afin d'estimer l'effet des cellules dédifférenciées de Bougainvillier sur le potentiel de renouvellement cellulaire de la lame basale de l'épiderme.

En effet, l'antigène Ki-67 fait partie des marqueurs de prolifération. Cet antigène est présent sur une protéine nucléaire de 360 kDa (numéro d'accession P46013) présent dans les cellules prolifératives. L'antigène Ki-67 fut décrit par Gerdes J *et al,* en 1983 après immunisation des souris par injection de noyaux de cellules de lymphome provenant d'un lymphome de Hodgkin (Gerdes J. et al, International Journal of Cancer, 1983, Volume 31, Issue 1, pages 13-20).

Il est présent au niveau du noyau des cellules prolifératives, en phases G₁, S, G₂ et M et absent sur les cellules en cycle de repos (phase G₀). Sa fonction précise n'est pas connue, cependant une participation au maintien du pouvoir prolifératif ou au contrôle du cycle cellulaire est suggérée.

L'antigène Ki-67 peut être détecté par un anticorps Ki-67, à savoir l'anticorps monoclonal MIB-1 (société R&D Systems) en immunohistochimie. En pratique, l'index de marquage par le Ki-67 représente le pourcentage de noyaux colorés par l'anticorps MI1-B.

### 5.2 Protocole

L'étude a été réalisée sur des épidermes humains reconstruits *in vitro,* de modèle RHEps, fabriqués par la société SkinEthic. Ils ont été répartis en 4 lots (n = 4, c'est-à-dire 4 épidermes par lot) :
**Lot 1** : 4 épidermes non traités ;
**Lot 2** : 4 épidermes traités avec la suspension de cellules végétales dédifférenciées et élicitées de Bougainvillier préparée ci-dessus à l'exemple 1, après dilution à 0,5 % avec du milieu nutritif liquide (milieu de Murashige et Skoog non gélosé), à raison de 2 µl/cm², à 37°C, sous une atmosphère à 5% CO₂ pendant 24 heures ;
**Lot 3** : 4 épidermes traités avec un extrait de safran à 1 % non encapsulé, c'est-à-dire avec du milieu nutritif liquide (milieu de Murashige et Skoog sans gélose) renfermant 1 % en masse d'extrait de safran, à raison de 2 µl/cm², à 37°C, sous une atmosphère à 5% CO₂ pendant 24 heures ;
**Lot 4 :** 4 épidermes traités avec la suspension de cellules végétales dédifférenciées et élicitées de Bougainvillier encapsulant un extrait de safran préparée ci-dessus à l'exemple 3, à raison de 2 µl/cm², à 37°C, sous une atmosphère à 5% CO₂ pendant 24 heures.

Les épidermes des lots 1 à 4 ont ensuite été fixés dans une solution de formaldéhyde à 10 %, puis inclus dans des blocs de paraffine. Des coupes verticales de 5 µm ont été réalisées au microtome. Chacune des coupes a ensuite été mise en contact avec une solution d'anticorps monoclonal MI1-B selon le protocole indiqué par le fournisseur R&D Systems. La révélation a été faite par la méthode peroxydase-anti peroxydase après démasquage antigénique par prétraitement à la chaleur également selon le protocole indiqué par R&D Systems. Le marquage par chromogène DAB (diamino-3,3'-benzidine tétrachlorhydrate) révèle en brun les sites nucléaires KI-67 de la fraction de cellules en croissance exprimées en phases G₁ et S (Phase de latence et synthèse de la cellule) ; G₂ (Phase de dédoublement des constituants cellulaire) et M (mitose).

L'index de prolifération KI-67 des épidermes traités par les cellules végétales dédifférenciées de Bougainvillier a été évalué par comptage des sites nucléaires colorés, à raison de 10 champs par lame, au microscope optique, grossissement x 250, comparativement aux coupes des épidermes témoins non traités. Les résultats obtenus sont présentés dans le tableau 1 ci-après :

**TABLEAU 1**

| | **Nombre de cellules colorées** | **Différence par rapport au témoin (%)** |
|---|---|---|
| **Epidermes Témoins (Lot 1)** | 16,5 ± 3 | - |
| **Epidermes traités par les cellules de Bougainvillier (Lot 2)** | 20,8 ± 0,8 ¹ | + 26 |
| **Epidermes traités par l'extrait de safran seul (Lot 3)** | 18,8 ± 0,5 | + 13 |
| **Epidermes traités par les cellules encapsulant l'extrait de safran (Lot 4)** | 22,4 ± 0,2 | + 35 |

| | | |
|---|---|---|
| ¹ : Significativement différent par rapport au témoin, p<0,05 (« *Wilcoxon Rank Sum Test* ») | | |

Ces résultats montrent que le traitement des épidermes par les cellules végétales dédifférenciées et élicitées de Bougainvillier induit déjà une augmentation de la prolifération cellulaire et par conséquent a un effet positif sur la régénération de l'épiderme. Ces essais montrent également que l'encapsulation de l'extrait de safran par les cellules dédifférenciées et élicitées de Bougainvillier a également induit une augmentation significative de la prolifération des cellules de la couche basale (+35 %) et que cette prolifération est beaucoup plus intense que celle observée après traitement des épidermes avec l'extrait de safran seul (Lot 3), ou qu'après traitement avec les cellules de Bougainvillier seules (Lot 2).

### EXEMPLE 6 : Evaluation de l'effet des cellules dédifférenciées et élicitées de Bougainvillier encapsulant un extrait de safran sur la régénération du derme

### 6.1 Principe du test

Le test est basé sur l'évaluation par immunomarquage en fluorescence du collagène I afin d'estimer l'effet des cellules dédifférenciées et élicitées de Bougainvillier encapsulant un extrait de safran sur la synthèse de collagène I et ainsi sur le potentiel de renouvellement du derme.

### 6.2 Protocole

L'étude a été réalisée sur des fibroblastes de derme humains NHDFs (Normal Human Dermal Fibroblasts ; origine : prépuce) à environ 40% de leur potentiel prolifératif et cultivés en monocouche en milieu DMEM (*Dulbecco's Modified Eagle Medium*) contenant du sérum de boeuf foetal (« *Fetal Bovine Sérum* » : FBS) et des antibiotiques (pénicilline/streptomycine).

Les cellules ont été maintenues dans une atmosphère humide à 37°C contenant 5% de CO₂.

Afin d'évaluer les effets sur la synthèse de collagène I, les fibroblastes NHDFs ont été ensemencés sur des lames de verre (compartiments de 0,7 cm²), 24 heures avant le début du traitement par les cellules dédifférenciées et élicitées de Bougainvillier encapsulant un extrait de safran. Cinq lots ont été constitués :
Lot 1 : témoin ne recevant aucun produit
Lot 2 : contrôle positif. Une solution de TGF-β1 à 20 ng/mL a été utilisée comme molécule de référence permettant d'augmenter la synthèse de collagène I au sein des fibroblastes NHDFs.
Lot 3 : les fibroblastes ont été traités avec 3% d'une suspension de cellules végétales élicités et dédifférenciées de Bougainvillier encapsulant un extrait de safran, préparées selon le même procédé que celui décrit ci-dessus à l'exemple 3.
Lot 4 : les fibroblastes ont été traités avec 1% d'une suspension de cellules végétales élicités et dédifférenciées de Bougainvillier encapsulant un extrait de safran, telles que préparées ci-dessus à l'exemple 3.
Lot 5 : les fibroblastes ont été traités avec 0,3% d'une suspension de cellules végétales élicités et dédifférenciées de Bougainvillier encapsulant un extrait de safran, préparées selon le même procédé que celui décrit ci-dessus à l'exemple 3.

Les cellules végétales élicitées de dédifférenciées de Bougainvillier encapsulant un extrait de safran ont été solubilisées dans le milieu de culture ne contenant pas de sérum et ont été mis en contact avec les fibroblastes durant 48 heures. Chaque condition a été réalisée en triplicat (n=3).

Au terme des traitements, les fibroblastes ont été fixés au formol puis rincés au PBS. Les lames ont ensuite été conservées à 4°C jusqu'au jour du marquage.

L'immuno-marquage en fluorescence du collagène I a été réalisé en incubant les lames avec un anticorps primaire spécifique et un anticorps secondaire conjugué à la fluorescéine. Le DAPI (4',6'-diamidino-2-phénylindole), une molécule fluorescente capable de lier l'ADN, a été utilisée pour marquer les noyaux. Les lames ont finalement été montées à l'alcool polyvinylique (produit vendu sous la dénomination commerciale Mowiol® par la société Aldrich) et stockées à 4°C.

La quantification a été réalisée sur la base de 3 photos par réplique prises à l'aide d'un microscope Leica ® (DM 2000, objectif 40x) et d'une caméra Leica ® (DFC420C).

Le marquage en immunofluorescence du collagène I a été quantifié à l'aide du logiciel QWin 3 ® (Leica) en utilisant le pixel comme unité de surface (1 pixel = 1 µm²). Chaque photo est constituée de 4751460 pixels. L'intensité moyenne du marquage a été déterminée pour chaque photo. Les noyaux ont été dénombrés grâce au logiciel *ImageJ* afin de rapporter la surface marquée et l'intensité moyenne du marquage au nombre de noyaux sur chaque photo. Ce rapport, permettant de déterminer l'intensité du marquage, est comparé à la valeur correspondant à la condition contrôle non traité.

### 6.3 Résultats

Les résultats sont présentés dans le tableau 2 ci-après.

**TABLEAU 2**

| Condition | Marquage en immunofluorescence du Collagène I | Différence par rapport au témoin (%) |
|---|---|---|
| Lot 1 (témoin) | 386497 ± 5 | - |
| Lot 2 | 1 833 434 ± 92 | +374 |
| Lot 3 | 677 881 ± 9 | + 75 |
| Lot 4 | 578 891 ± 53 | + 50 |
| Lot 5 | 534 275 ± 23 | + 38 |

Ces résultats montrent que le traitement des fibroblastes par les cellules végétales dédifférenciées et élicitées de Bougainvillier encapsulant un extrait de safran, aux différentes concentrations testées 3%, 1% et 0,3%, induit une augmentation du marquage en immunofluroescence du collagène I (respectivement + 75%, +50% et +38% par rapport au témoin).

On peut donc en déduire que l'application de cellules dédifférenciées et élicitées de Bougainvillier encapsulant un extrait de safran permet d'augmenter la synthèse de collagène I, et ainsi a un effet positif sur la régénération du derme.

### EXEMPLE 7 : Mise en évidence des propriétés des cellules végétales de Bougainvillier conformes à l'invention sur la viabilité et la respiration cellulaires

Dans cet exemple on a évalué les propriétés de la suspension de cellules végétales dédifférenciées et élicitées encapsulant un extrait de safran telle que préparée ci-dessus à l'exemple 3, sur la viabilité et la respiration cellulaires, ainsi que sur le métabolisme énergétique.

### 7.1 Principe du test

L'activité de la suspension de cellules végétales de l'exemple 3 sur le métabolisme cellulaire et respiratoire a été évaluée par la métabolisation du glucose par les cellules de l'épiderme dans des conditions d'hypoxie. Les conditions d'hypoxie *in vitro* entrainent des altérations profondes des fonctions électromécaniques cellulaires, accompagnés d'une augmentation de la production de lactate, d'une chute des teneurs en adénosine triphosphate (ATP) et en adénosine diphosphate (ADP), et d'une fuite de lactate déshydrogénase (LDH). La réoxygénation des cellules hypoxiées (à un stable réversible) normalise la perte de lactate, entraine une resynthèse d'ATP et une atténuation de la libération de LDH. En effet, la respiration cellulaire est une réaction chimique d'oxydoréduction ayant lieu dans les mitochondries et qui fournit l'énergie nécessaire à une cellule pour fonctionner. La respiration cellulaire fait intervenir le cycle de Krebs et nécessite un carburant qui est principalement le glucose et qui est issu de la glycolyse anaérobie, ainsi qu'un comburant qui est le dioxygène (O₂). Cette réaction produit principalement du dioxyde de carbone (CO₂) et de l'eau.

Ainsi pour démontrer ces effets au niveau de l'épiderme, la respiration et la viabilité des cellules ont été évaluées par le dosage du relargage du ¹⁴CO₂ après traitement des cellules des épidermes par le [¹⁴C]-glucose-6-phosphate (SIGMA ALDRICH) selon un protocole permettant de suivre un processus cellulaire dynamique et connu sous l'appellation anglophone « *pulse*/*chase ».*

### 7.2 Protocole du test

Le test a été effectué *in vitro* sur épidermes humains reconstruits (« *Reconstructed Human Epidermis* », RHE) modèle RHEps, de surface 0,5 cm², vendus par la société SkinEthic ®. Ils ont été répartis en 3 lots (n = 4, c'est-à-dire 4 épidermes par lot) :
**Lot 1** : épidermes témoin négatif ne recevant aucun produit et aucun traitement ;
**Lot 2** : épidermes témoin positif ne recevant aucun produit et un traitement d'asphyxie pendant 24 heures ;
**Lot 3 :** 4 épidermes traités avec la suspension de cellules végétales dédifférenciées et élicitées de Bougainvillier encapsulant un extrait de safran préparée ci-dessus à l'exemple 3 et préalablement diluée à 0,5 % avec du milieu de culture Murashige et Skoog non gélosé, en application topique pendant 24 heures sur les épidermes, à raison de 2 µl par cm² pendant 24 heures et traitement d'asphyxie pendant 24 heures.

Il est à noter que dans les expériences ci-dessus, l'asphyxie des épidermes a été obtenue par incubation des épidermes, sous vide, dans des boîtes fermées hermétiquement pendant toute la durée du traitement (24 heures).

### 7.3 Résultats

Les résultats obtenus sont présentés dans le tableau 3 ci-après, la quantité de ¹⁴CO₂ libéré étant exprimée en coups par minute (cpm) :

**TABLEAU 3**

| | **Quantité de ¹⁴CO₂ libéré (cpm)** | **Différence par rapport au témoin (%)** |
|---|---|---|
| **Epidermes Témoin négatif (Lot 1)** | 1845 ± 112 | - |
| **Epidermes Témoin positif (asphyxie) (Lot 2)** | 1098 ± 105 ¹ | - 40 |
| **Epidermes traités par les cellules encapsulant l'extrait de safran + asphyxie (Lot 3)** | 1496 ± 93 ² | + 36 |

| | | |
|---|---|---|
| ¹ : Significativement différent rapport au témoin négatif p<0,05 (« *Wilcoxon Rank Sum Test* ») ² : Significativement différent par rapport au témoin positif p<0,05 (« *Wilcoxon Rank Sum Test* ») | | |

Ces résultats révèlent que l'application des cellules de Bougainvillier conformes à l'invention entraine une augmentation significative du relargage du ¹⁴CO₂, dans les conditions d'asphyxie par rapport au témoin positif. On peut donc en déduire que l'application de cellules dédifférenciées et élicitées de Bougainvillier encapsulant un extrait de safran permet d'augmenter significativement le métabolisme énergétique cellulaire, et ainsi a un effet positif sur la viabilité des cellules constituant l'épiderme.

### EXEMPLE 8 : Mise en évidence de la libération progressive de l'extrait après application des cellules de Bougainvillier conformes à l'invention

Dans cet exemple, on a étudié la cinétique de libération de l'extrait de safran encapsulé dans les cellules végétales dédifférenciées et élicitées telles que préparées ci-dessus à l'exemple 3, après application sur l'épiderme, comparativement à l'application d'un extrait de safran seul, c'est-à-dire non encapsulé dans des cellules végétales dédifférenciées et élicités de Bougainvillier.

### 8.1 Principe du test

Tout comme à l'exemple précédent, la cinétique de libération de l'extrait de safran a été étudiée et évaluée par le dosage du relargage du ¹⁴CO₂ après traitement des cellules des épidermes par le [¹⁴C]-glucose-6-phosphate.

### 8.2 Protocole du test

Le test a été effectué *in vitro* sur épidermes humains reconstruits (« *Reconstructed Human Epidermis* », RHE) modèle RHEps, de surface 0,5 cm², vendus par la société SkinEthic ®. Ils ont été répartis en 12 lots (n = 4, c'est-à-dire 4 épidermes par lot) :

### Traitement des épidermes pendant 2 heures :

**Lot T2-1** : épidermes témoin négatif ne recevant aucun produit et aucun traitement ;
**Lot T2-2 :** épidermes témoin positif ne recevant aucun produit et un traitement d'asphyxie pendant 2 heures ;
**Lot T2-3** : épidermes traités avec une solution à 1 % en masse d'extrait de safran dans du milieu nutritif liquide (milieu de Murashige et Skoog sans gélose), en application topique pendant 2 heures sur les épidermes, à raison de 2 µl par cm² et traitement d'asphyxie pendant 2 heures ;
**Lot T2-4 :** épidermes traités avec la suspension de cellules végétales dédifférenciées et élicitées de Bougainvillier encapsulant un extrait de safran préparée ci-dessus à l'exemple 3 et préalablement diluée à 0,5 % avec du milieu de culture Murashige et Skoog non gélosé, en application topique pendant 2 heures sur les épidermes, à raison de 2 µl par cm² et traitement d'asphyxie pendant 2 heures.

### Traitement des épidermes pendant 6 heures :

**Lot T6-1** : épidermes témoin négatif ne recevant aucun produit et aucun traitement ;
**Lot T6-2 :** épidermes témoin positif ne recevant aucun produit et un traitement d'asphyxie pendant 6 heures ;
**Lot T6-3** : épidermes traités avec une solution à 1 % en masse d'extrait de safran dans du milieu nutritif liquide (milieu de Murashige et Skoog sans gélose), en application topique pendant 6 heures sur les épidermes, à raison de 2 µl par cm² et traitement d'asphyxie pendant 6 heures ;
**Lot T6-4 :** épidermes traités avec la suspension de cellules végétales dédifférenciées et élicitées de Bougainvillier encapsulant un extrait de safran préparée ci-dessus à l'exemple 3 et préalablement diluée à 0,5 % avec du milieu de culture Murashige et Skoog non gélosé, en application topique pendant 6 heures sur les épidermes, à raison de 2 µl par cm² et traitement d'asphyxie pendant 6 heures.

### Traitement des épidermes pendant 12 heures :

**Lot T12-1** : épidermes témoin négatif ne recevant aucun produit et aucun traitement ;
**Lot T12-2** : épidermes témoin positif ne recevant aucun produit et un traitement d'asphyxie pendant 12 heures ;
**Lot T12-3** : épidermes traités avec une solution à 1 % en masse d'extrait de safran dans du milieu nutritif liquide (milieu de Murashige et Skoog sans gélose), en application topique pendant 12 heures sur les épidermes, à raison de 2 µl par cm² et traitement d'asphyxie pendant 12 heures ;
**Lot T12-4** : épidermes traités avec la suspension de cellules végétales dédifférenciées et élicitées de Bougainvillier encapsulant un extrait de safran préparée ci-dessus à l'exemple 3 et préalablement diluée à 0,5 % avec du milieu de culture Murashige et Skoog non gélosé, en application topique pendant 12 heures sur les épidermes, à raison de 2 µl par cm² et traitement d'asphyxie pendant 12 heures.

### 8.3 Résultats

Les résultats obtenus sont présentés dans le tableau 4 ci-après, la quantité de ¹⁴CO₂ libéré étant exprimée en coups par minutes (cpm) :

**TABLEAU 4**

| | **Quantité de ¹⁴CO₂ libéré (cpm)** | | | | | |
|---|---|---|---|---|---|---|
| | **2 heures d'incubation** | **Différence par rapport au témoin (%)** | **6 heures d'incubation** | **Différence par rapport au témoin (%)** | **12 heures d'incubation** | **Différence par rapport au témoin (%)** |
| **Epidermes Témoin négatif** | 1532 ± 42 | - | 1598 ± 35 | - | 1612 ± 87 | - |
| **Epidermes Témoin positif (asphyxie)** | 1385 ± 12 ¹ | - 9 | 1245 ± 28 ¹ | - 22 | 1095 ± 53 ¹ | - 32 |
| **Epidermes traités à l'extrait de safran non encapsulé + asphyxie** | 1395 ± 29 | ns | 1352 ± 57 | ns | 1295 ± 49 ² | + 18 |
| **Epidermes traités par les cellules encapsulant l'extrait de safran + asphyxie** | 1662 ± 61 ² | + 20 | 1556 ± 21 ² | + 25 | 1445 ± 51 ² | + 32 |

| | | | | | | |
|---|---|---|---|---|---|---|
| ¹ : Significativement différent par rapport au témoin négatif p<0,05 (« *Wilcoxon Rank Sum Test* ») ² : Significativement différent par rapport au témoin positif p<0,05 (« *Wilcoxon Rank Sum Test* ») | | | | | | |

Ces résultats montrent que l'extrait de safran appliqué seul, c'est-à-dire, sans être encapsulé dans les cellules végétales de Bougainvillier induit déjà un effet significatif mais faible (+ 18) sur le relargage du CO₂ (Lot T12-3) lorsque le temps d'incubation est de 12 heures. Par contre, lorsque l'extrait de safran est encapsulé dans les cellules de Bougainvillier, on observe un effet significatif sur le relargage du CO₂, dès 2 heures d'incubation (+20 %), avec une amplification au cours du temps (+ 25 % après 6 heures d'incubation et + 32 % après 12 heures d'incubation) révélant une libération progressive de l'extrait de safran au coeur de l'épiderme.

### EXEMPLE 9 : Mise en évidence de l'effet des cellules végétales de Bougainvillier conformes à l'invention sur l'épaisseur de l'épiderme

Dans cet exemple on a évalué les effets de la suspension de cellules végétales dédifférenciées et élicitées encapsulant un extrait de safran telle que préparée ci-dessus à l'exemple 3, sur l'épaisseur de l'épiderme.

### Protocole du test

Le test a été effectué *in vitro* sur épidermes humains reconstruits (« *Reconstructed Human Epidermis* », RHE) modèle RHEps, de surface 0,5 cm², vendus par la société SkinEthic ®. Ils ont été répartis en 2 lots (n = 4, c'est-à-dire 4 épidermes par lot) :
**Lot 1** : 4 épidermes témoins non traités,
**Lot 2** : 4 épidermes traités avec la suspension de cellules végétales dédifférenciées et élicitées de Bougainvillier encapsulant un extrait de safran préparée ci-dessus à l'exemple 3 et préalablement diluée à 0,5 % avec du milieu de culture Murashige et Skoog non gélosé, en application topique pendant 24 heures sur les épidermes, à raison de 2 µl par cm² pendant 24 heures.

A la fin du traitement, c'est-à-dire après contact avec les cellules de Bougainvillier, les épidermes du lot 2, ainsi que les épidermes non traités du lot 1, ont été fixés dans une solution de formaldéhyde à 10 %, puis inclus dans des blocs de paraffine. Des coupes verticales de 4 µm ont été réalisées au microtome puis colorées à l'hématoxyline/éosine selon les techniques connues de l'homme du métier.

L'épaisseur des épidermes a été déterminée par un analyseur d'image (Zeiss) sous un microscope optique (Zeiss), avec un grossissement de 40 fois.

Les résultats obtenus sont présentés dans le tableau 5 ci-après :

**TABLEAU 5**

| | **Epaisseur moyenne (µm)** | **Différence par rapport au témoin (%)** |
|---|---|---|
| **Epidermes Témoins (Lot 1)** | 137 ± 12 | - |
| **Epidermes traités par les cellules encapsulant l'extrait de safran (Lot 2)** | 162 ± 9 ¹ | + 18 |

| | | |
|---|---|---|
| ¹ : Significativement différent par rapport au témoin, p<0,05 (« *Wilcoxon Rank Sum Test* ») | | |

Ces résultats mettent en évidence que l'application des cellules dédifférenciées et élicitées de Bougainvillier encapsulant un extrait de safran sur les épidermes entrainent une augmentation significative de leur épaisseur.

L'ensemble des résultats présentés dans les exemples qui précèdent démontre que les cellules végétales dédifférenciées et élicitées de bougainvillier encapsulant l'extrait de safran conformes à l'invention induisent une stimulation cutanée. Cette dernière a été observée au niveau de tous les compartiments épidermiques, d'une façon étalée dans le temps. En effet, les cellules de Bougainvillier encapsulant l'extrait de safran ont induit une augmentation de l'épaisseur de l'épiderme et un renforcement de la densité par une nette prolifération des cellules basales. Ces activités sont dues à la stimulation du métabolisme cellulaire et respiratoire des cellules de l'épiderme.

Par ailleurs, et bien que ces effets aient été démontrés en choisissant un extrait de safran à titre d'ingrédient actif, ils sont généralisables à tout autre ingrédient actif ayant des propriétés intéressantes pour les soins de la peau dans la mesure où les cellules dédifférenciées et élicitées de Bougainvillier mises en oeuvre dans cette invention présentent en tant que telles des propriétés de régénération du derme et de l'épiderme et permettent d'encapsuler et de libérer tout type d'ingrédient actif de façon progressive au coeur des différentes couches de l'épiderme et ainsi de potentialiser leurs effets.

### EXEMPLE 10 : Sérum pour le soin de la peau

Par les techniques usuelles, on a préparé un sérum pour le soin de la peau ayant la composition massique indiquée ci-après.
- Glutamate diacétate tetrasodique 0,07 g
- Butylèneglycol d'origine végétale vendu sous la dénomination commerciale Zemea ® par la société DKSH 5,00 g
- Gomme de xanthane 0,07 g
- Hydroxyéthyl cellulose 0,30 g
- Lécithine hydrogénée 1,00 g
- Triglycérides d'acides caprique et caprylique vendus sous la dénomination commerciale Myritol ® 318 par la société BASF 3,00 g
- Stéaroyl-2-lactylate de sodium 0,20 g
- Glycéryl undécylénate 0,50 g
- Tetraisopalmitate d'ascorbyle 0,10 g
- Sel de sodium de l'acide dihydroacétique 0,10 g
- Hyaluronate de sodium 0,15 g
- Tréhalose 1,00 g
- Fucose 0,50 g
- Suspension de cellules végétales encapsulant un extrait de safran telle que préparée à l'exemple 3 ci-dessus 2,00 g
- Amidon de tapioca (manioc) 0,35 g
- Parfum 0,10 g
- Eau déminéralisée qsp 100,00 g

Ce sérum est destiné à raffermir et régénérer la peau et peut être utilisé une à deux fois par jour.

### EXEMPLE 11 : Crème anti-âge

Par les techniques usuelles, on a préparé une crème anti-âge ayant la composition massique suivante :
- Glutamate diacétate tetrasodique 0,10 g
- Butylèneglycol d'origine végétale vendu sous la dénomination commerciale Zemea ® par la société DKSH 5,00 g
- Glycérine vendue sous la dénomination commerciale Glycérine Bio par la société IES 8,00 g
- Bétaïne 2,00 g
- Hydroxyéthyl cellulose 0,50 g
- Déhydroacétate de sodium 0,15 g
- Emulsionnant lamellaire naturel composé de phospholipides et de lipides végétaux, vendu sous la dénomination commerciale Biophilic ® H par la société Lucas Meyer Cosmetics 4,00 g
- Glycéryl stéarate citrate 0,80 g
- Squalane d'origine végétale vendu sous la dénomination commerciale Phytosqualane par la société Sophim 5,00 g
- Triglycérides d'acides caprique et caprylique vendus sous la dénomination commerciale Myritol ® 318 par la société BASF 7,00 g
- Alcool butylique 1,00 g
- Alcool béhénylique 0,75 g
- Huile de Macadamia 2,00 g
- Huile de karité oléique vendue sous la dénomination commerciale Beurre de *Vitellaria nilotica* par la société Olvea 4,00 g
- Glycéryl undécylénate 0,50 g
- Tetraisopalmitate d'ascorbyle 0,25 g
- Suspension de cellules végétales encapsulant un extrait de safran telle que préparée à l'exemple 3 ci-dessus 5,00 g
- Géranyl géranyl propanol vendu sous la dénomination commerciale Juvinity® par la société Sederma 2,00 g
- Parfum 0,25 g
- Hyaluronate de sodium 0,20 g
- Tréhalose 1,00 g
- Mélange de palmitoyl oligopeptide et de palmitoyl-tetrapeptide-7 vendu sous la dénomination commerciale Matrixyl ® 3000 par la société Sederma Corp. 3,00 g
- Eau déminéralisée qsp 100,00 g

Cette crème anti-âge peut être utilisée une à deux fois par jour par application sur les zones de la peau à traiter.

### EXEMPLE 12 : Lotion tonique rafraîchissante

Par les techniques usuelles, on a préparé une lotion tonique rafraichissante ayant la composition massique suivante :
- Butylèneglycol d'origine végétale vendu sous la Dénomination commerciale Zemea ® par la société DKSH 5,00 g
- Acide galacturonique 0,10 g
- Acide anisique 0,10 g
- Mélange d'acide lévulinique et de lévulinate de sodium vendu sous la dénomination commerciale Dissolvine ® GL 38 par la société AkzoNobel 0,30 g
- Sel de sodium de l'acide pyrrolidone carboxylique (Sodium PCA) 0,50 g
- Eau de menthe poivrée (société Herbarom) 10,00 g
- Eau de Mélisse (société Herbarom) 10,00 g
- Eau de Tilleul (société Herbarom) 10,00 g
- Eau de Rosa Centifolia (société Herbarom) 25,00 g
- Extrait de fleurs de Souci 1,00 g
- Suspension de cellules végétales encapsulant un extrait de safran telle que préparée à l'exemple 3 ci-dessus 1,00 g
- Eau de Romarin (société Herbarom) 3,00 g
- Eau déminéralisée qsp 100,00 g

Cette lotion tonique rafraichissante peut être utilisée une à deux fois par jour par application sur les zones de la peau à traiter.

## Revendications

1. Utilisation de cellules végétales dédifférenciées et élicitées de Bougainvillier pour l'encapsulation d'au moins un ingrédient actif.

2. Cellules végétales dédifférenciées et élicitées de Bougainvillier se présentant sous la forme de vésicules comprenant une membrane végétale formée d'une bicouche lipidique délimitant un volume interne renfermant une phase encapsulée, **caractérisées en ce qu'**au moins un ingrédient actif est présent dans ladite phase encapsulée et/ou au sein de ladite bicouche lipidique.

3. Cellules selon la revendication 2, **caractérisées en ce que** l'ingrédient actif est choisi parmi un extrait de plancton, un extrait de maca, un extrait de pétales de coquelicot, un extrait de safran, une combinaison d'extraits d'anchuse, de coquelicot et de passiflore, un extrait de graines de mimosa, un extrait de pétales de souci, un extrait de barbitamao, un extrait de kigelia africana et un extrait d'avoine.

4. Cellules selon la revendication 2 ou 3, **caractérisées en ce que** l'ingrédient actif est un extrait de safran.

5. Cellules selon l'une quelconque des revendications 2 à 4, **caractérisées en ce que** la quantité en ingrédient actif encapsulé varie de 0,01 à 5 % en masse par rapport à la masse totale des cellules dédifférenciées et élicitées de Bougainvillier.

6. Cellules selon l'une quelconque des revendications 2 à 5, **caractérisées en ce que** la phase encapsulée est une phase aqueuse.

7. Suspension de cellules végétales de Bougainvillier dans la glycérine, **caractérisée en ce que** lesdites cellules végétales sont des cellules dédifférenciées et élicitées de Bougainvillier telle que définies à l'une quelconque des revendications 2 à 6.

8. Suspension selon la revendication 7, **caractérisée en ce que** la quantité de cellules dédifférenciées et élicitées de Bougainvillier au sein de ladite suspension varie de 20 à 30 % en masse par rapport à la masse totale de ladite suspension.

9. Procédé de préparation de cellules dédifférenciées et élicitées de Bougainvillier encapsulant au moins un ingrédient actif telles que définies à l'une quelconque des revendications 2 à 6, ledit procédé comprenant les étapes suivantes :
1) la préparation de cellules dédifférenciées par culture *in vitro* en milieu de culture d'explants de Bougainvillier,
2) l'élicitation des cellules dédifférenciées obtenues à l'étape précédente dans le milieu de culture,
3) l'extraction des cellules dédifférenciées et élicitées obtenues ci-dessus à l'étape précédente,
ledit procédé étant **caractérisé en ce qu'**il comprend en outre une étape 4) d'encapsulation d'au moins un ingrédient actif dans des cellules dédifférenciées et élicitées, cette étape étant réalisée par incubation des cellules dédifférenciées et élicitées obtenues ci-dessus à l'étape 3) avec une solution ou une dispersion d'au moins un ingrédient actif dans un milieu liquide, ladite incubation.

10. Procédé selon la revendication 9, **caractérisé en ce que** le milieu liquide utilisable pour effectuer l'incubation des cellules dédifférenciées et élicitées avec le ou les ingrédients actifs est choisi parmi les milieux utilisés à l'étape 1) dudit procédé pour la culture *in vitro* desdites cellules.

11. Composition cosmétique à application topique **caractérisée en ce qu'**elle comprend, à titre d'ingrédient, des cellules végétales dédifférenciées et élicitées de Bougainvilliers encapsulant au moins un ingrédient actif telles que définies à l'une quelconque des revendications 2 à 6, et au moins un support cosmétiquement acceptable.

12. Composition selon la revendication 11, **caractérisée en ce qu'**elle comprend de 0,001 à 10 % en masse d'une suspension de cellules végétales dédifférenciées de Bougainvillier dans la glycérine telle que définie à l'une quelconque des revendications 7 ou 8.

13. Composition selon la revendication 11 ou 12, **caractérisée en ce qu'**elle se présente sous forme de gel, d'émulsion, d'émulsion biphasique huile-dans-eau ou eau-dans-huile, d'huile corporelle, de masque, de pommade, d'onguent, de lotion ou de solution concentrée.

14. Composition selon l'une quelconque des revendications 11 à 13, **caractérisée en ce qu'**elle comprend en outre des agents de protection contre les rayons ultraviolets ou des pigments formant écran anti-ultraviolet.

15. Procédé cosmétique non thérapeutique pour les soins de la peau, **caractérisé en ce qu'**il consiste à appliquer sur les zones de la peau concernées au moins une composition cosmétique telle que définie à l'une quelconque des revendications 11 à 14.

16. Utilisation non thérapeutique d'une composition cosmétique à application topique comprenant des cellules végétales dédifférenciées et élicitées de Bougainvillier encapsulant au moins un ingrédient actif et telles que définies à l'une quelconque des revendications 11 à 14 pour les soins de la peau.

## Patentansprüche

1. Verwendung dedifferenzierter und aktivierter Bougainvillea-Pflanzenzellen für das Einkapseln mindestens eines aktiven Inhaltsstoffs.

2. Dedifferenzierte und aktivierte Bougainvillea-Pflanzenzellen in Form von Vesikeln, umfassend eine pflanzliche Membran, gebildet von einer Lipid-Doppelschicht, die ein inneres Volumen begrenzt, das eine eingekapselte Phase einschließt, **dadurch gekennzeichnet, dass** mindestens ein aktiver Inhaltsstoff in der eingekapselten Phase und/oder in der Lipid-Doppelschicht vorhanden ist.

3. Zellen nach Anspruch 2, **dadurch gekennzeichnet, dass** der aktive Inhaltsstoff aus einem Extrakt von Plankton, einem Extrakt von Maca, einem Extrakt von Blütenblättern des Mohns, einem Extrakt von Safran, einer Kombination von Extrakten von Ochsenzunge, Mohn und Passionsblume, einem Extrakt von Samen von Mimose, einem Extrakt von Blütenblättern von Ringelblume, einem Extrakt von Barbitamao, einem Extrakt von Kigelia africana und einem Extrakt von Hafer ausgewählt ist.

4. Zellen nach Anspruch 2 oder 3, **dadurch gekennzeichnet, dass** der aktive Inhaltsstoff ein Extrakt von Safran ist.

5. Zellen nach einem der Ansprüche 2 bis 4, **dadurch gekennzeichnet, dass** die Menge an eingekapseltem aktivem Inhaltsstoff von 0,01 bis 5 Ma% in Bezug zur Gesamtmasse der dedifferenzierten und aktivierten Bougainvillea-Zellen schwankt.

6. Zellen nach einem der Ansprüche 2 bis 5, **dadurch gekennzeichnet, dass** die eingekapselte Phase eine wässrige Phase ist.

7. Suspension von Bougainvillea-Pflanzenzellen in Glycerin, **dadurch gekennzeichnet, dass** die Pflanzenzellen dedifferenzierte und aktivierte Bougainvillea-Zellen nach einem der Ansprüche 2 bis 6 sind.

8. Suspension nach Anspruch 7, **dadurch gekennzeichnet, dass** die Menge dedifferenzierter und aktivierter Bougainvillea-Zellen innerhalb der Suspension von 20 bis 30 Ma% in Bezug zur Gesamtmasse der Suspension schwankt.

9. Verfahren zur Herstellung dedifferenzierter und aktivierter Bougainvillea-Zellen, die mindestens einen aktiven Inhaltsstoff nach einem der Ansprüche 2 bis 6 einkapseln, wobei das Verfahren die folgenden Schritte umfasst:
1)das Herstellen dedifferenzierter Zellen durch in vitro-Kultur im Bougainvilleaexplantat-Kulturmilieu,
2) das Aktivieren der in vorangehendem Schritt erhaltenen dedifferenzierten Zellen im Kulturmilieu,
3) das Extrahieren der oben in vorangehendem Schritt erhaltenen dedifferenzierten und aktivierten Zellen,
wobei das Verfahren **dadurch gekennzeichnet ist, dass** es ferner einen Schritt 4) des Einkapselns mindestens eines aktiven Inhaltsstoffs in dedifferenzierten und aktivierten Zellen umfasst, wobei dieser Schritt durch Inkubieren der in obigem Schritt 3) erhaltenen dedifferenzierten und aktivierten Zellen mit einer Lösung oder eine Dispersion von mindestens einem aktiven Inhaltsstoff in einem flüssigen Milieu, der Inkubation, durchgeführt wird.

10. Verfahren nach Anspruch 9, **dadurch gekennzeichnet, dass** das flüssige Milieu, das für die Durchführung der Inkubation der dedifferenzierten und aktivierten Zellen mit dem oder den aktiven Inhaltsstoffen verwendbar ist, aus dem Milieus ausgewählt ist, die in Schritt 1) des Verfahrens für die in vitro-Kultur der Zellen verwendet werden.

11. Kosmetische Zusammensetzung zur topischen Anwendung, **dadurch gekennzeichnet, dass** sie als Inhaltsstoff dedifferenzierte und aktivierte Bougainvillea-Pflanzenzellen umfasst, die mindestens einen aktiven Inhaltsstoff nach einem der Ansprüche 2 bis 6 und mindestens einen kosmetisch akzeptablen Träger einkapseln.

12. Zusammensetzung nach Anspruch 11, **dadurch gekennzeichnet, dass** sie 0,001 bis 10 Ma% einer Suspension dedifferenzierter und aktivierter Bougainvillea-Pflanzenzellen in Glycerin nach einem der Ansprüche 7 oder 8 umfasst.

13. Zusammensetzung nach Anspruch 11 oder 12, **dadurch gekennzeichnet, dass** sie in Form von Gel, Emulsion, Zweiphasen-ÖI-in-Wasser- oder -Wasser-in-ÖI-Emulsion, Körperöl, Maske, Pomade, Salbe, Lotion oder konzentrierter Lösung vorliegt.

14. Zusammensetzung nach einem der Ansprüche 11 bis 13, **dadurch gekennzeichnet, dass** sie ferner Schutzmittel vor ultravioletten Strahlen oder Pigmente umfasst, die einen UV-Schutzschild bilden.

15. Nicht therapeutisches kosmetisches Verfahren zur Hautpflege, **dadurch gekennzeichnet, dass** es darin besteht, auf die betroffenen Bereiche der Haut mindestens eine kosmetische Zusammensetzung nach einem der Ansprüche 11 bis 14 aufzutragen.

16. Nicht therapeutische Verwendung einer kosmetischen Zusammensetzung zur topischen Anwendung, umfassend dedifferenzierte und aktivierte Bougainvillea-Zellen, die mindestens einen aktiven Inhaltsstoff nach einem der Ansprüche 11 bis 14 einkapseln, zur Hautpflege.

## Claims

1. Use of dedifferentiated, elicited plant cells of Bougainvillea for encapsulating at least one active ingredient.

2. Dedifferentiated, elicited cells of Bougainvillea in the form of vesicles comprising a plant membrane formed of a lipid bilayer delimiting an inner volume containing an encapsulated phase, **characterized in that** at least one active ingredient is contained in said encapsulated phase and/or in said lipid bilayer.

3. The cells according to claim 2, **characterized in that** the active ingredient is selected from among a plankton extract, maca extract, poppy petal extract, saffron extract, a combination of Anchusa, poppy and Passiflora extracts, mimosa seed extract, marigold petal extract, Barbitamao extract, Kigelia Africana extract and an oat extract.

4. The cells according to claim 2 or 3, **characterized in that** the active ingredient is a saffron extract.

5. The cells according to any of claims 2 to 4, **characterized in that** the amount of encapsulated active ingredient varies from 0.01 to 5 weight % relative to the total weight of the dedifferentiated, elicited cells of Bougainvillea.

6. The cells according to any of claims 2 to 5, **characterized in that** the encapsulated phase is an aqueous phase.

7. Suspension of Bougainvillea plant cells in glycerine, **characterized in that** said plant cells are dedifferentiated, elicited cells of Bougainvillea such as defined in any of claims 2 to 6.

8. The suspension according to claim 7, **characterized in that** the amount of dedifferentiated, elicited cells of Bougainvillea in said suspensions varies from 20 to 30 weight % relative to the total weight of said suspension.

9. Method for preparing dedifferentiated, elicited cells of Bougainvillea encapsulating at least one active ingredient such as defined in any of claims 2 to 6, said method comprising the following steps:
1) preparing dedifferentiated cells via *in vitro* culture in a culture medium of Bougainvillea explants;
2) eliciting the dedifferentiated cells obtained at the preceding step in the culture medium;
3) extracting the dedifferentiated, elicited cells obtained above at the preceding step,
said method being **characterized in that** it further comprises a step 4) to encapsulate at least one active ingredient in dedifferentiated, elicited cells, this step being performed by incubating the dedifferentiated, elicited cells obtained above at step 3) with a solution or dispersion of at least one active ingredient in a liquid medium, said incubation.

10. The method according to claim 9, **characterized in that** the liquid medium that can be used to incubate the dedifferentiated, elicited cells with the active ingredient(s) is selected from among the media used at step 1) of said method for *in vitro* culture of said cells.

11. Cosmetic composition for topical application, **characterized in that** as active ingredient it comprises dedifferentiated, elicited plant cells of Bougainvillea encapsulating at least one active ingredient such as defined in any of claims 2 to 6, and at least one acceptable cosmetic carrier.

12. The composition according to claim 11, **characterized in that** it comprises from 0.001 to 10 weight % of a suspension of dedifferentiated Bougainvillea plant cells in glycerine such as defined in any of claims 7 or 8.

13. The composition according to claim 11 or 12, **characterized in that** it is in the form of a gel, emulsion, two-phase oil-in-water or water-in-oil emulsion, body oil, mask, ointment, unguent, lotion or concentrated solution.

14. The composition according to any of claims 11 to 13, **characterized in that** it further comprises agents protecting against ultraviolet radiation or pigments forming an anti-ultraviolet screen.

15. Non-therapeutic cosmetic method for skin care, **characterized in that** it consists of applying to the skin regions concerned at least one cosmetic composition such as defined in any of claims 11 to14.

16. Non-therapeutic use of a cosmetic composition for topical application comprising dedifferentiated, elicited plant cells of Bougainvillea encapsulating at least one active ingredient and such as defined in any of claims 11 to 14, for skin care.
